# EUROPEAN PATENT APPLICATION

(11) **EP 4 825 360 A1**
(43) Date of publication of application: **21.10.2026**
(21) Application number: 24903574.2
(22) Date of filing: 04.12.2024
(51) Int. Cl.: A61K 6/891, A61K 6/833

(54) **DENTAL COMPOSITION**

(30) Priority: 12.12.2023 JP 2023209300
(71) Applicant: Kuraray Noritake Dental Inc., Kurashiki-shi, Okayama 710-0801 (JP); JFE Chemical Corporation, Tokyo 111-0051 (JP)
(72) Inventor: MATSUURA, Ryo, Tainai-shi, Niigata 959-2653 (JP); ISHIGURO, Eri, Tainai-shi, Niigata 959-2653 (JP); KITAHARA, Yoshinori, Tokyo 111-0051 (JP)
(74) Representative: D Young & Co LLP
(86) International application number: PCT/JP2024/042807
(87) International publication number: WO 2025/126917

(57) **Abstract**

Provided is a dental composition that has a large depth of photocuring, and is capable of providing a cured product that has a high mechanical strength, a small polymerization shrinkage stress, and a high surface hardness. The dental composition contains a monomer (A), a polymerization initiator (B), and a filler (C), the monomer (A) contains a monomer (A-1) represented by the following formula (1) and a monomer (A-2) that is other than the monomer (A-1), and the dental composition contains the filler (C) in an amount of 50 to 90% by mass based on 100% by mass of the dental composition, in which the symbols in the formula (1) are as defined in the description:

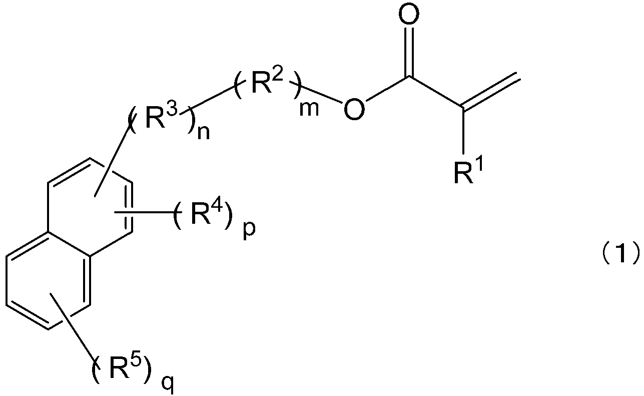

## Description

### Technical Field

The present invention relates to a dental composition.

### Background Art

Known dental compositions include a dental filling and repairing material used for filling a cavity formed in a tooth and repairing the cavity, such as a dental composite resin, a self-adhesive dental composite resin, and dental cement, and also a dental core build-up material for packing or covering a devitalized tooth having a large defect to restore the tooth to an abutment tooth form.

In order for a cured product of the dental composition to have sufficient strength, a dental composition containing a polymerizable monomer having a bisphenol A skeleton, represented by 2,2-bis[4-(meth)acryloyloxypolyethoxyphenyl]propane, has been widely used.

It has also been known to add a filler to the dental composition for the purpose of adjusting the paste property, imparting X-ray opacity to the cured product, enhancing the mechanical strength of the cured product, and the like.

However, the ordinary polymerizable monomer having a bisphenol A skeleton has a refractive index that is not sufficiently high, and therefore the ordinary dental composition containing the polymerizable monomer having a bisphenol A skeleton has a large difference in refractive index from the filler, which is likely to cause scattering of light, and thus is likely to decrease the depth of photocuring. The decreased depth of photocuring causes sufficient curing of the dental composition to be difficult, resulting in a problem of difficulty in enhancing the mechanical strength of the cured product. Accordingly, there is a demand of a dental composition that has a large depth of photocuring and provides a cured product having a high mechanical strength.

On the other hand, dental materials containing a polymerizable monomer that does not contain a bisphenol A skeleton have also been proposed (see, for example, PTLs 1 to 8).

PTL 9 describes a resin composition for stereolithography used for a dental occlusal splint and the like, which indeed does not relate to a dental filling and repairing material or a dental core build-up material, and the resin composition for stereolithography contains an α,β-unsaturated double bond group-containing compound having a cyclic structure (a) having a nitrogen atom, a (meth)acrylate ester compound that has a prescribed atmospheric boiling point, has a carbocyclic ring, and does not have a nitrogen-containing heterocyclic group, and a photopolymerization initiator. However, the technical issues to be solved by the invention described in PTL 9 include odor, lithographic capability, toughness, and water resistance, and scattering of light in the case where a filler is contained in the resin composition is not considered. PTL 9 does not describe or suggest the securement of a sufficiently large depth of photocuring while adding a filler to the resin composition at a high proportion.

### Citation List

### Patent Literatures

PTL 1: JP 2013-60373 A
PTL 2: JP 5008852 B
PTL 3: WO 2019/107322
PTL 4: JP 5819415 B
PTL 5: JP 2010-506931 A
PTL 6: JP 6458034 B
PTL 7: WO 2020/218446
PTL 8: WO 2021/132463
PTL 9: WO 2020/129736

### Summary of Invention

### Technical Problem

However, the dental compositions having been proposed still have a room for improvement, and it is the actual situation that it is difficult to have a large depth of photocuring and provide a cured product that is good in all the mechanical strength, the polymerization shrinkage stress, and the surface hardness. Accordingly, there is still a demand of a novel and useful dental composition that is different from the ordinary compositions.

Under the circumstances, a problem to be solved by the present invention is to provide a dental composition that is capable of providing a cured product having a high mechanical strength, a small polymerization shrinkage, a large depth of photocuring, and a high surface hardness.

### Solution to Problem

As a result of various investigations by the present inventors, it has been found that the problem can be solved by a dental composition containing a monomer having a particular structure and a prescribed amount of a filler, and thus the present invention has been completed. Specifically, the present invention encompasses the following inventions.
[1] A dental composition containing a monomer (A), a polymerization initiator (B), and a filler (C),
   the monomer (A) containing a monomer (A-1) represented by the following formula (1) and a monomer (A-2) that is other than the monomer (A-1),
   the dental composition containing the filler (C) in an amount of 50 to 90% by mass based on 100% by mass of the dental composition: 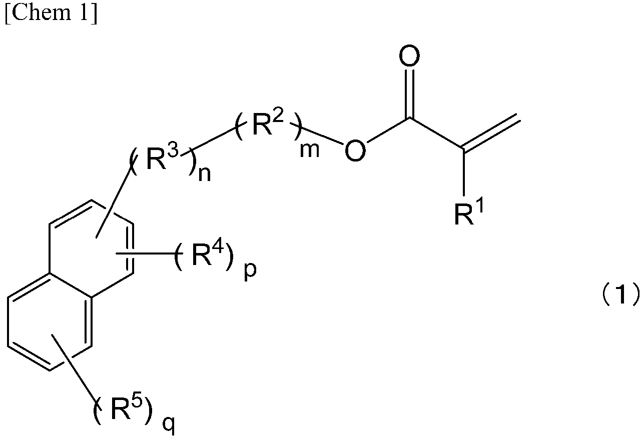 in which,
   R¹ represents a hydrogen atom or a methyl group, R² represents a divalent hydrocarbon group, R³ represents an oxygen atom or a sulfur atom,
   R⁴ and R⁵ each independently represent a halogen atom, an alkyl group, or an alkoxy group, m represents 0 or 1, n represents 0 or 1, p represents 0 to 3, and q represents 0 to 4,
   provided that
   in the case where m represents 0, n represents 0,
   in the case where p represents 2 or more, the multiple atoms or groups represented by R⁴ are the same atom or group or different atoms or groups, and
   in the case where q represents 2 or more, the multiple atoms or groups represented by R⁵ are the same atom or group or different atoms or groups.
[2] The dental composition according to the item [1], in which the dental composition contains the monomer (A-1) in an amount of 1 to 80 parts by mass per 100 parts by mass in total of the monomer (A-1) and the monomer (A-2).
[3] The dental composition according to the item [1] or [2], in which the monomer (A-1) is a monomer (A-1a) represented by the following formula (2): 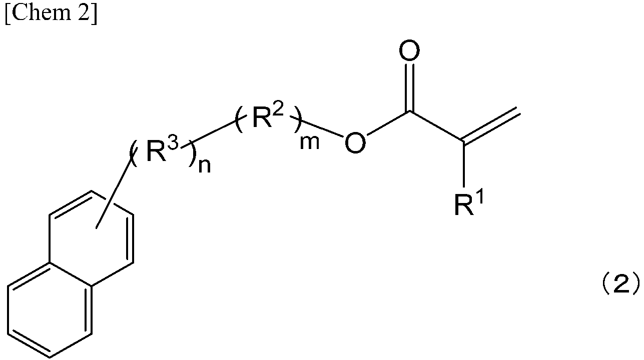 in which, R¹, R², R³, m, and n have the same meanings as defined in the formula (1).
[4] The dental composition according to any one of the items [1] to [3], in which the monomer (A-1) is 1-naphthylmethyl (meth)acrylate.
[5] The dental composition according to any one of the items [1] to [4], in which the monomer (A-2) contains a monomer having a urethane bond.
[6] The dental composition according to any one of the items [1] to [5], in which the monomer (A-2) contains a monomer having a (meth)acryloyl group.
[7] The dental composition according to any one of the items [1] to [6], in which the dental composition has a content of a monomer having a bisphenol A skeleton of more than 0% by mass and 30% by mass or less, or does not contain a monomer having a bisphenol A skeleton.
[8] The dental composition according to any one of the items [1] to [7], in which the dental composition is a dental composite resin.
[9] The dental composition according to any one of the items [1] to [7], in which the dental composition is dental cement.
[10] The dental composition according to any one of the items [1] to [7], in which the dental composition is a self-adhesive dental composite resin.
[11] The dental composition according to any one of the items [1] to [7], in which the dental composition is a dental core build-up material.

### Advantageous Effects of Invention

The present invention provides a dental composition that has a large depth of photocuring, and is capable of providing a cured product that has a high mechanical strength, a small polymerization shrinkage stress, and a high surface hardness.

### Description of Embodiments

The present invention is described with reference to embodiments below.

In the description herein, the upper limit values and the lower limit values of the numerical ranges (e.g., the contents of the components, and the values and the properties calculated from the components) each can be optionally combined. In the description herein, the numerical values of the symbols in the formulae can also be optionally combined.

Specifically, in the description herein, the upper limit values and the lower limit values described in a stepwise manner for the numerical ranges each can be independently combined. For example, with reference to the description "preferably 10 to 90, and more preferably 30 to 60" for the same item, a range of "10 to 60" can be derived from the "preferred lower limit value (10)" and the "more preferred upper limit value (60)".

For the numerical ranges, for example, based on the description "preferably 10 to 90, and more preferably 30 to 60", only the lower limit value can be defined as "10 or more" or "30 or more" with no particular definition of the upper limit value. Similarly, only the upper limit value can be defined as "90 or less" or "60 or less" with no particular definition of the lower limit value.

A numerical range simply defined as "10 to 90" means a range of 10 or more and 90 or less unless otherwise indicated.

As similar to as described above, for example, with reference to the description "preferably 10 or more, and more preferably 30 or more" and the description "preferably 90 or less, and more preferably 60 or less" for the same item, a range of "10 or more and 60 or less" can be derived from the "preferred lower limit value (10)" and the "more preferred upper limit value (60)". As similar to as described above, only the lower limit value can be defined as "10 or more" or "30 or more", and similarly, only the lower limit value can be defined as "90 or less" or "60 or less".

In the description herein, the expression "(meth)acrylic" is used as meaning encompassing both "methacrylic" and "acrylic". The same is applied to the similar expressions, such as "(meth)acrylate", "(meth)acrylate ester", "(meth)acrylamide", and "(meth)acryloyloxy".

In the description herein, the expression "(meth)acrylic based monomer" or "(meth)acrylic based compound" is used as meaning encompassing both "(meth)acrylate ester based compound" and "(meth)acrylamide and a derivative thereof".

### [Dental Composition]

The dental composition according to an embodiment of the present invention is a dental composition containing a monomer (A), a polymerization initiator (B), and a filler (C), the monomer (A) contains a monomer (A-1) represented by the following formula (1) and a monomer (A-2) that is other than the monomer (A-1), and the dental composition contains the filler (C) in an amount of 50 to 90% by mass based on 100% by mass of the dental composition.

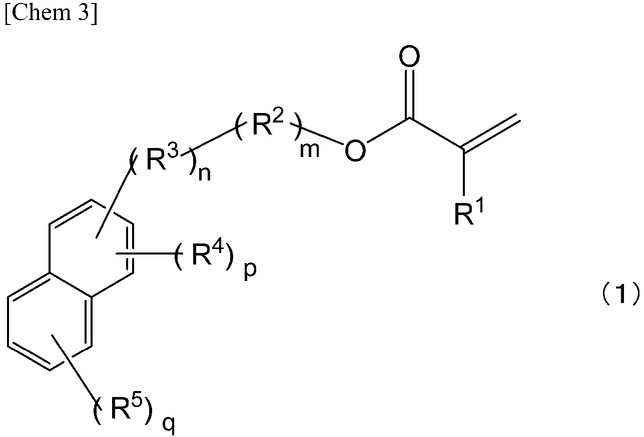

The mechanism that allows the dental composition to have a high depth of photocuring, and allows the cured product thereof to have a high mechanical strength, a low polymerization shrinkage stress, and a high surface hardness is presumed to be the following, but not limited thereto.

The compound represented by the formula (1) (which may be hereinafter referred to as a "compound (1)") has a relatively rigid skeleton, and therefore it is considered that the cured product of the dental composition exhibits a high mechanical strength and a high surface curability, combined with the presence of the filler contained in a high proportion. Furthermore, the compound (1) is monofunctional, and therefore it is presumed the polymerization shrinkage stress in curing the dental composition is reduced. Moreover, the compound (1) generally has a high refractive index due to the naphthalene skeleton having two aromatic rings bonded to each other, and the difference in refractive index between the monomer component and the filler can be reduced. Therefore, it is considered that the scattering due to the difference in refractive index between the monomer component and the filler can be reduced, and eventually a dental composition having a large depth of photocuring can be obtained.

The meanings of the symbols in the formula (1) are described later.

### <Monomer (A)>

The monomer (A) contains a monomer (A-1) represented by the formula (1) and a monomer (A-2) that is other than the monomer (A-1). Hereinafter, the monomer (A-1) represented by the formula (1) may be referred simply to as a "monomer (A-1)", and the monomer (A-2) that is other than the monomer (A-1) may be referred simply to as a "monomer (A-2)".

### (Monomer (A-1))

The monomer (A-1) is at least one kind of a compound represented by the formula (1). The monomer (A-1) may be a single compound represented by the formula (1), or may be a mixture of multiple kinds of compounds represented by the formula (1).

In the formula (1), R¹ represents a hydrogen atom or a methyl group, and preferably represents a methyl group.

In the formula (1), R² represents a divalent hydrocarbon group, and preferably represents a methylene group.

In the formula (1), R³ represents an oxygen atom or a sulfur atom, and preferably represents an oxygen atom.

In the formula (1), R⁴ and R⁵ each independently represent a halogen atom, an alkyl group, or an alkoxy group, and preferably represents a halogen atom or an alkyl group.

In the formula (1), m represents 0 or 1, and n represents 0 or 1, in which in the case where m represents 0, n represents 0.

In the case where m represents 1, and n represents 0, one of the bonding sites of the group represented by R² is bonded to the carbon atom on the naphthalene skeleton in the formula (1).

In the case where m and n represent 0, the other bonding site of the oxygen atom bonded to the carbonyl group in the formula (1) is bonded to the carbon atom on the naphthalene skeleton in the formula (1).

In the formula (1), it is preferred that m represents 1, and n represents 0.

In the formula (1), p represents 0 to 3, and q represents 0 to 4.

In the case where p represents 2 or more, the multiple atoms or groups represented by R⁴ are the same atom or group or different atoms or groups.

In the case where q represents 2 or more, the multiple atoms or groups represented by R⁵ are the same atom or group or different atoms or groups.

In the formula (1), it is preferred that p represents 0, and q represents 0.

The monomer (A-1) is preferably a monomer (A-1a) represented by the following formula (2) from the standpoint of facilitating the enhancement of the mechanical strength and the surface hardness.

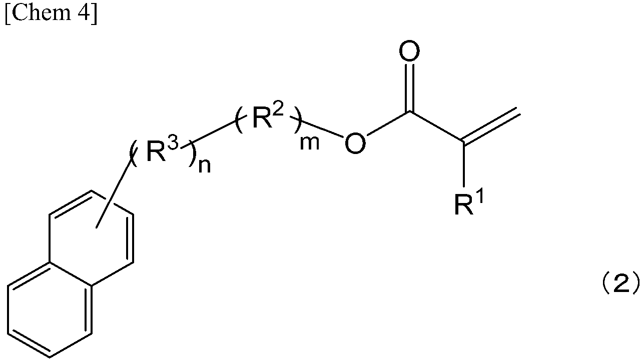

In the formula (2), R¹, R², R³, m, and n have the same meanings as defined in the formula (1), and preferred ones thereof are also the same.

The monomer (A-1) is more preferably a monomer (A-1b) represented by the following formula (3) from the standpoint of facilitating the reduction of the polymerization shrinkage stress while preventing the decrease of the depth of photocuring.

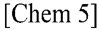

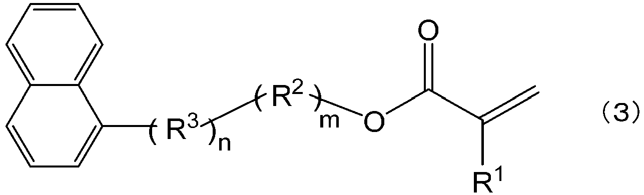

In the formula (3), R¹, R², R³, m, and n have the same meanings as defined in the formula (1).

The monomer (A-1) is further preferably a monomer (A-1c) represented by the following formula (4) from the standpoint of further facilitating the reduction of the polymerization shrinkage stress while preventing the decrease of the depth of photocuring.

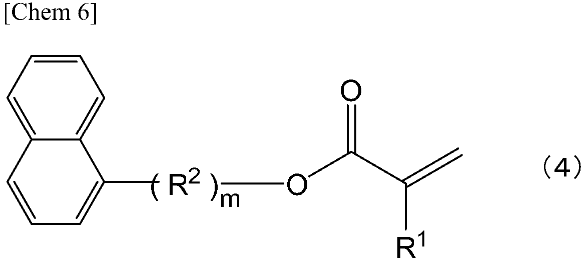

In the formula (4), R¹, R², and m have the same meanings as defined in the formula (1).

The monomer (A-1) is more preferably 1-naphthylmethyl (meth)acrylate or 2-naphthylmethyl (meth)acrylate from the standpoint of facilitating the enhancement of the mechanical strength and the surface hardness, and is further preferably 1-naphthylmethyl (meth)acrylate, and particularly preferably 1-naphthylmethyl methacrylate, from the standpoint of further facilitating the reduction of the polymerization shrinkage stress while preventing the decrease of the depth of photocuring.

### (Monomer (A-2))

The monomer (A-2) is a compound that is other than the monomer (A-1), i.e., a polymerizable monomer having a structure that is different from the compound (1).

The monomer (A-2) preferably contains a monomer having a urethane bond, and is more preferably a polyfunctional polymerizable monomer having a urethane bond, from the standpoint of the mechanical strength of the cured product.

The monomer (A-2) preferably contains a monomer having a (meth)acryloyl group from the standpoint of the curability, and more preferably contains multiple (meth)acryloyl groups from the standpoint of further enhancing the curability.

The monomer (A-2) is described in detail below.

Examples of the monomer (A-2) include a monofunctional polymerizable monomer having one polymerizable group and a polyfunctional polymerizable monomer having multiple polymerizable groups. The monomer (A-2) preferably contains a polyfunctional polymerizable monomer from the standpoint of achieving the good curability.

Examples of the monofunctional polymerizable monomer include a monofunctional (meth)acrylate based polymerizable monomer and a monofunctional (meth)acrylamide based polymerizable monomer.

Examples of the monofunctional (meth)acrylate based polymerizable monomer include 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, methyl (meth)acrylate, ethyl (meth)acrylate, propyl (meth)acrylate, isopropyl (meth)acrylate, n-butyl (meth)acrylate, sec-butyl (meth)acrylate, tert-butyl (meth)acrylate, isobutyl (meth)acrylate, n-hexyl (meth)acrylate, cyclohexyl (meth)acrylate, lauryl (meth)acrylate, cetyl (meth)acrylate, stearyl (meth)acrylate, isobornyl (meth)acrylate, tetrahydrofurfuryl (meth)acrylate, benzyl (meth)acrylate, phenyl (meth)acrylate, o-phenylphenyl (meth)acrylate, m-phenylphenyl (meth)acrylate, p-phenylphenyl (meth)acrylate, pentabromobenzyl (meth)acrylate, pentachlorophenyl (meth)acrylate, anthracenyl (meth)acrylate, anthracenylmethyl (meth)acrylate, (o-phenylphenoxy)methyl (meth)acrylate, (m-phenylphenoxy)methyl (meth)acrylate, (p-phenylphenoxy)methyl (meth)acrylate, (o-phenylphenoxy)ethyl (meth)acrylate, (m-phenylphenoxy)ethyl (meth)acrylate, (p-phenylphenoxy)ethyl (meth)acrylate, (o-phenylphenoxy)propyl (meth)acrylate, (m-phenylphenoxy)propyl (meth)acrylate, (p-phenylphenoxy)propyl (meth)acrylate, (o-phenylphenoxy)butyl (meth)acrylate, (m-phenylphenoxy)butyl (meth)acrylate, (p-phenylphenoxy)butyl (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, 6-(p-phenoxyphenyl)hexyl (meth)acrylate, piperidinyl (meth)acrylate, tetramethylpiperidinyl (meth)acrylate, pentamethylpiperidinyl (meth)acrylate, (meth)acrylic acid, potassium (meth)acrylate, magnesium (meth)acrylate, zinc (meth)acrylate, 2,3-dibromopropyl (meth)acrylate, 3-(meth)acryloyloxypropyltrimethoxysilane, and 11-(meth)acryloyloxyundecyltrimethoxysilane. One kind of these compounds may be used alone, or two or more kinds thereof may be used in combination.

Examples of the monofunctional (meth)acrylamide based polymerizable monomer include (meth)acrylamide, N-(meth)acryloylmorpholine, N,N-dimethyl(meth)acrylamide, N,N-diethyl(meth)acrylamide, N,N-di-n-propyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-hydroxyethyl (meth)acrylamide, N,N-bis(2-hydroxyethyl)acrylamide, N-(meth)acryloylcarbazole, N-methyl-N-phenyl(meth)acrylamide, N-(meth)acryloylmorpholine, N-piperidinylacrylamide, N-tetramethylpiperidinylacrylamide, N-2-methylpiperidinylacrylamide, N-3-methylpiperidinylacrylamide, and N-4-methylpiperidinylacrylamide. One kind of these compounds may be used alone, or two or more kinds thereof may be used in combination.

The structure of the polyfunctional polymerizable monomer is not particularly limited, and is preferably a polyfunctional polymerizable monomer containing a urethane bond from the standpoint of the excellent curability. Examples of the polyfunctional polymerizable monomer include an aromatic compound based polyfunctional polymerizable monomer and an aliphatic compound based polyfunctional polymerizable monomer, in which an aromatic compound based polyfunctional polymerizable monomer is preferred from the standpoint of facilitating the achievement of the good curability.

The polyfunctional polymerizable monomer containing a urethane bond may be a compound having a urethane bond introduced to be adjacent to the (meth)acrylate group, and examples thereof include a urethanated (meth)acrylate based polymerizable monomer having at least one kind of a structure selected from the group consisting of a polyester, a polycarbonate, a polyurethane, a polyether, a poly(conjugated diene), and a hydrogenated poly(conjugated diene) (which may be hereinafter referred to as a "polymer backbone") in one molecule, and a urethanated (meth)acrylate based polymerizable monomer having no polymer backbone, in which among these, a urethanated (meth)acrylate based polymerizable monomer having a polymer backbone is preferred, and a compound having an aromatic ring in a repeating unit constituting the polymer backbone is more preferred.

The urethanated (meth)acrylate based polymerizable polyfunctional monomer having a polymer backbone can be readily synthesized, for example, by subjecting a polyol having the polymer backbone, a compound having an isocyanate group (-NCO), and a (meth)acrylate compound having a hydroxy group (-OH) to addition reaction. The urethanated (meth)acrylate based polyfunctional polymerizable monomer having a polymer backbone can also be readily synthesized by subjecting a (meth)acrylate compound having a hydroxy group and a lactone or an alkylene oxide to ring-opening addition reaction, and then subjecting the resulting compound having a hydroxy group at one end and a compound having an isocyanate group to addition reaction.

The polyol having the polymer backbone is not particularly limited, as long as having the aforementioned structure, and examples of the polyester include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of a β-propiolactone, a polymer of γ-butyrolactone, a polymer of δ-valerolactone, a polymer of ε-caprolactone, and copolymers thereof. Examples of the polycarbonate include a polycarbonate derived from an aliphatic diol having 2 to 12 carbon atoms, a polycarbonate derived from bisphenol A, and a polycarbonate derived from an aliphatic diol having 2 to 12 carbon atoms and bisphenol A. Examples of the polyurethane include a polymer of an aliphatic diol having 2 to 12 carbon atoms and a diisocyanate having 1 to 12 carbon atoms. Examples of the polyether include polyethylene glycol, polypropylene glycol, polybutylene glycol, and a poly(1-methylbutylene glycol). Examples of the poly(conjugated diene) and the hydrogenated poly(conjugated diene) include 1,4-polybutadiene, 1,2-polybutadiene, polyisoprene, poly(butadiene-isoprene), poly(butadienestyrene), poly(isoprene-styrene), polyfarnesene, and hydrogenated products thereof.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylate compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, N-hydroxyethyl (meth)acrylamide, N,N-bis(2-hydroxyethyl) (meth)acrylamide, 2-hydroxy-3-acryloyloxypropyl (meth)acrylate, 2,2-bis[4-{3-(meth)acryloyloxy-2-hydroxypropoxy}phenyl]propane, 1,2-bis[3-(meth)acryloyloxy-2-hydroxypropoxy]ethane, pentaerythritol tri(meth)acrylate, and dipentaerythritol tri- or tetra(meth)acrylate.

The addition reaction of the compound having an isocyanate group and the (meth)acrylate compound having a hydroxy group can be performed by a known method with no particular limitation.

Examples of the urethanated (meth)acrylate based polyfunctional polymerizable monomer having the polymer backbone include a reaction product of an optional combination of the polyol having at least one kind of a structure selected from the group consisting of a polyester, a polycarbonate, a polyurethane, a polyether, a poly(conjugated diene), and a hydrogenated poly(conjugated diene), the compound having an isocyanate group, and the (meth)acrylate compound having a hydroxy group. The urethanated (meth)acrylate based polyfunctional polymerizable monomer having the polymer backbone used may be a commercially available product. Examples of the commercially available product include "UFC-01" (available from Kyoeisha Chemical Co., Ltd.) and "EBECRYL 8465" (available from Daicel-Allnex Ltd.), both of which are a urethane acrylate.

The weight average molecular weight (Mw) of the urethanated (meth)acrylate based polyfunctional polymerizable monomer having the polymer backbone is preferably 500 to 50,000, more preferably 750 to 30,000, and further preferably 1,000 to 15,000, from the standpoint of the viscosity and the strength. The weight average molecular weight (Mw) in the present invention means a polystyrene conversion weight average molecular weight obtained by gel permeation chromatography (GPC).

Examples of the urethanated (meth)acrylate based polyfunctional polymerizable monomer having no polymer backbone include 2,2,4-trimethylhexamethylene bis(2-carbamoyloxyethyl) dimethacrylate (trivial name: "UDMA"), N,N-(2,2,4-trimethylhexamethylene)bis[2-(aminocarboxy)propane-1,3-diol] tetra(meth)acrylate, a phenyl glycidyl ether acrylate hexamethylene diisocyanate urethane prepolymer, a pentaerythritol triacrylate hexamethylene diisocyanate urethane prepolymer, a pentaerythritol triacrylate toluene diisocyanate urethane prepolymer, a pentaerythritol triacrylate isophorone diisocyanate urethane prepolymer, and a dipentaerythritol pentaacrylate hexamethylene diisocyanate urethane prepolymer.

Examples of the aromatic compound based polyfunctional polymerizable monomer include 2,2-bis((meth)acryloyloxyphenyl)propane, 2,2-bis[4-(3-acryloyloxy)-2-hydroxypropolxyphenyl)propane, 2,2-bis[4-(3-methacryloyloxy)-2-hydroxypropolxyphenyl)propane (trivial name: "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytetraethoxyphenyl)propane, 2-(4-(meth)acryloyloxydipropoxyphenyl)-2-(4-(meth)acryloyloxytetraethoxyphenyl)propane, 2,2-bis(4-(meth)acryloyloxypropoxyphenyl)propane, 2,2-bis(4-(meth)acryloyloxyisopropoxyphenyl)propane, 2,2-bis(4-methacryloyloxypolyethoxyphenyl)propane (having an average addition molar number of ethoxy group of 2.6, trivial name: "D-2.6E"), and 1,4-bis(2-(meth)acryloyloxyethyl) pyromellitate.

One kind of these compounds may be used alone, or two or more kinds thereof may be used in combination.

However, it is preferred that the dental composition has a content of a monomer having a bisphenol A skeleton of more than 0% by mass and 30% by mass or less, or does not contain a monomer having a bisphenol A skeleton, from the standpoint of preventing the decrease of the depth of photocuring. In the case where the dental composition contains a monomer having a bisphenol A skeleton, the content thereof is more preferably more than 0% by mass and 25% by mass or less, and further preferably more than 0% by mass and 20% by mass or less.

Therefore, as for the monomers having a bisphenol A skeleton among the monomers described above (i.e., the compounds exemplified as the aromatic compound based polyfunctional polymerizable monomer except for 1,4-bis(2-(meth)acryloyloxyethyl)pyromellitate), it is preferred that the content of the monomers is more than 0% by mass and 30% by mass or less based on the total amount of the dental composition, or the monomers are not contained in the dental composition.

Examples of the aliphatic compound based polyfunctional polymerizable monomer that is a bifunctional polymerizable monomer include glycerol di(meth)acrylate, ethylene glycol di(meth)acrylate, diethylene glycol di(meth)acrylate, triethylene glycol di(meth)acrylate, tetraethylene glycol di(meth)acrylate, pentaethylene glycol di(meth)acrylate, hexaethylene glycol di(meth)acrylate, heptaethylene glycol di(meth)acrylate, octaethylene glycol di(meth)acrylate, nonaethylene glycol di(meth)acrylate, polyethylene glycol di(meth)acrylate, propylene glycol di(meth)acrylate, dipropylene glycol di(meth)acrylate, tripropylene glycol di(meth)acrylate, tetrapropylene glycol di(meth)acrylate, pentapropylene glycol di(meth)acrylate, hexapropylene glycol di(meth)acrylate, heptapropylene glycol di(meth)acrylate, octapropylene glycol di(meth)acrylate, nonapropylene glycol di(meth)acrylate, polypropylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and tricyclodecane dimethanol di(meth)acrylate.

Examples of the aliphatic compound based polyfunctional polymerizable monomer that is a trifunctional or higher polymerizable monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, and 1,7-diacryloyloxy-2,2,6,6-tetra(meth)acryloyloxymethyl-4-oxaheptane.

One kind of these compounds may be used alone, or two or more kinds thereof may be used in combination.

In the case where the monomer (A-2) contains at least one kind of the urethanated (meth)acrylate based polyfunctional polymerizable monomer, the aliphatic compound based polyfunctional (meth)acrylate based polymerizable monomer, and the aromatic compound based polyfunctional (meth)acrylate based polymerizable monomer, the content of the polyfunctional (meth)acrylate based polymerizable monomers is preferably 20 to 90 parts by mass, more preferably 30 to 80 parts by mass, and further preferably 40 to 70 parts by mass, per 100 parts by mass in total of the monomer (A-2).

In the case where the monomer (A-2) contains the monofunctional (meth)acrylate based polymerizable monomer or the monofunctional (meth)acrylamide based polymerizable monomer, the content of the monofunctional (meth)acrylate based polymerizable monomer or the monofunctional (meth)acrylamide based polymerizable monomer is preferably 0 to 60 parts by mass, more preferably 5 to 55 parts by mass, and further preferably 10 to 50 parts by mass, per 100 parts by mass in total of the monomer (A-2).

It is preferred that the monomer (A-2) does not contain an α,β-unsaturated double bond group-containing compound having a cyclic structure containing a nitrogen atom, or the content of the compound is more than 0% by mass and 10% by mass or less.

Examples of the α,β-unsaturated double bond group-containing compound having a cyclic structure containing a nitrogen atom include a compound that has a cyclic structure containing one or more nitrogen atom as a hetero atom in the structure thereof, and contains an α,β-unsaturated double bond group.

Examples of the cyclic structure containing one or more nitrogen atom include a cyclic structure containing only one or more nitrogen atom as a hetero atom on the cyclic structure, a cyclic structure containing both a nitrogen atom and an oxygen atom as hetero atoms on the cyclic structure, and a cyclic structure containing both a nitrogen atom and a sulfur atom as hetero atoms on the cyclic structure.

Examples of the α,β-unsaturated double bond group include a (meth)acryloyl group, a vinyl group, an N-vinyl group, a vinyl ester group, a vinyl silyl group, a vinyl ether group, an allyl group, and a methallyl group.

Examples of the α,β-unsaturated double bond group-containing compound containing only one nitrogen atom as a hetero atom on the cyclic structure include a (meth)acrylate ester compound having a nitrogen atom-containing monocyclic structure, such as pentamethylpiperidinyl (meth)acrylate (alternative name: (1,2,2,6,6-pentamethyl-4-piperidyl (meth)acrylate), tetramethylpiperidinyl (meth)acrylate (alternative name: (meth)acrylic acid 2,2,6,6-tetramethyl-4-piperidyl), piperidinyl (meth)acrylate (alternative name: (meth)acrylic acid-4-piperidyl), 4-(pyrimidin-2-yl)piperazin-1-yl (meth)acrylate; a (meth)acrylamide compound having a nitrogen atom-containing monocyclic structure, such as N-(meth)acryloylpyrrolidine, N-(meth)acryloylpiperidine, piperidyl(meth)acrylamide (alternative name: N-(piperidin-4-yl)(meth)acrylamide), N-(meth)acryloyl-2-methylpiperidine, and N-(meth)acryloyl-2,2,6,6-tetramethylpiperidine; a vinyl group-containing compound having a nitrogen atom-containing 5-membered ring, such as 1-vinylpyrrole, 2-vinylpyrrole, 2-methyl-5-vinyl-1H-pyrrole, 1-vinyl-2-imidazoline, 2-vinyl-2-imidazoline, 1-vinyl-2-methyl-2-imidazoline, 4,5-dihydro-2-vinyl-1H-imidazole, 1-vinylimidazole, 2-vinyl-1H-imidazole, 1-vinyl-1H-pyrazole, 1-vinyl-3,5-dimethyl-1H-pyrazole, and 3-methyl-5-phenyl-1-vinylpyrazole; a vinyl group-containing compound having a nitrogen atom-containing 6-membered ring, such as 2-vinylpiperazine, 4-vinylpiperazine, 1-benzyl-2-vinylpiperazine, 1-benzyl-3-vinylpiperazine, 1,4-dimethyl-3-vinylpiperazine, 2-vinylpyridine, 3-vinylpyridine, 4-vinylpyridine, 6-methyl-2-vinylpyridine, 2-vinylpyrazine, 2-methyl-5-vinylpyrazine, 2-methyl-6-vinylpyrazine, 2,5-dimethyl-3-vinylpyrazine, 2-vinylpyrimidine, 2-vinylpyridazine, 2-vinyl-4,6-diamino-1,3,5-triazine, 6-vinyl-1,3,5-dimethyl-2,4-diamine, and 3-vinyl-1,2,4,5-tetrazine; a vinyl group-containing compound having an acyl group and a nitrogen atom-containing 6-membered ring, such as vinyl nicotinoylacetate, vinyl nicotinoylpropionate, vinyl nicotinoylbutyrate, vinyl nicotinoylvalerate, vinyl nicotinoylhexanoate, vinyl nicotinoyldecanoate, vinyl nicotinoyldodecanoate, vinyl isonicotinoylacetate, vinyl isonicotinoylpropionate, vinyl isonicotinoylbutyrate, vinyl isonicotinoylvalerate, vinyl isonicotinoylhexanoate, vinyl isonicotinoyldecanoate, and vinyl isonicotinoyldodecanoate; a vinyl group-containing compound having a nitrogen atom-containing polycyclic structure, such as 1-vinylindole, 1-vinyl-2-methyl-1H-indole, 1-vinylisoindole, 1-vinyl-1H-benzimidazole, 2-vinyl-1H-benzimidazole, 2-vinyl-5,6-dimethyl-1H-benzimidazole, 1-vinylindazole, 2-vinylquinoline, 4-vinylquinoline, 2-vinylisoquinoline, 2-vinylisoxaline, 2-vinylquinoxaline, 2-vinylquinazoline, 2-vinylcinnoline, 1-vinylcarbazole, and N-vinylcarbazole; a compound containing a nitrogen atom-containing monocyclic structure and two or more vinyl groups, such as 1-methyl-4,5-divinyl-1H-imidazole, 1,1'-divinyl-2,2'-bi(1H-imidazole), 2,3-divinylpyridine, 2,4-divinylpyridine, 2,5-divinylpyridine, and 2,6-divinylpyridine; a (meth)allyl group-containing compound having a nitrogen atom-containing cyclic structure, such as 1-(meth)allyl-1H-imidazole (1-allyl-1H-imidazole and 1-methallyl-1H-imidazole are generically expressed as "1-(meth)allyl-1H-imidazole", the same applies hereinafter), 1-(meth)allyl-2-methyl-1H-imidazole, 1-(meth)allyl-3-methyl-1H-imidazol-3-ium, 1-(meth)allyl-3-ethyl-1H-imidazol-3-ium, 4-(meth)allyl-3,5-dimethyl-1H-pyrazole, 5-bromo-1-1-(meth)allyl-1H-pyrazole, 1-(meth)allylpiperazine, 5-(1-methylpropyl)-5-(meth)allylpyrimidine, 5-(meth)allyl-5-isopropylpyrimidine, 1-(meth)allyl-5,5-diethylpyrimidine, 2-(meth)allylpyridine, 4-(meth)allylpyridine, and 3,6-dihydro-3-(meth)allylpyridine; and a (meth)allyl group-containing compound having a nitrogen atom-containing polycyclic structure, such as 2-(meth)allyl-1H-indole, 3-(meth)allyl-1H-indole, 1-(meth)allyl-1H-benzimidazole, 2-(meth)allylindazole, 1-(meth)allyl-3-methyl-1H-indazole, 1-(meth)allyl-4-methyl-1H-indazole, N-(meth)allylquinolin-4-amine, di(meth)allylquinoline, 3-phenyl-4-(meth)allylisoquinoline, 1,2-di(meth)allyl-1,2-dihydroisoquinoline, and 9-(meth)allyl-9H-carbazole.

Examples of the α,β-unsaturated double bond group-containing compound containing only two or more nitrogen atoms as hetero atoms on the cyclic structure include a (meth)acrylate ester compound having a nitrogen atom-containing polycyclic structure, such as 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, 2-(2'-hydroxy-5'-(meth)acryloyloxypropylphenyl)-2H-benzotriazole, 2-(2'-hydroxy-5'-(meth)acryloyloxypropylphenyl)-5-chloro-2H-benzotriazole, 2-(2'-hydroxy-3'-tert-butyl-5'-(meth)acryloyloxyethylphenyl)-2H-benzotriazole, and 2-(2'-hydroxy-3'-tert-butyl-5'-(meth)acryloyloxyethylphenyl)-5-chloro-2H-benzotriazole; and a (meth)acrylate ester compound having a nitrogen atom-containing 6-membered ring, such as 2,4-diphenyl-6-[2-hydroxy-4-{2-(meth)acryloyloxyethoxy}]-s-triazine, 2,4-bis(2-methylphenyl)-6-[2-hydroxy-4-{2-(meth)acryloyloxyethoxy}]-s-triazine, 2,4-bis(2-methoxyphenyl)-6-[2-hydroxy-4-{2-(meth)acryloyloxyethoxy}]-s-triazine, 2,4-bis(2-ethylphenyl)-6-[2-hydroxy-4-{2-(meth)acryloyloxyethoxy}]-s-triazine, 2,4-bis(2-ethoxyphenyl)-6-[2-hydroxy-4-{2-(meth)acryloyloxyethoxy}]-s-triazine, 2,4-bis(2,4-dimethylphenyl)-6-[2-hydroxy-4-{2-(meth)acryloyloxyethoxy}]-s-triazine, 2,4-bis(2,4-diethoxyphenyl)-6-[2-hydroxy-4-{2-(meth)acryloyloxyethoxy}]-s-triazine, and 2,4-bis(2,4-diethylphenyl)-6-[2-hydroxy-4-{2-(meth)acryloyloxyethoxy}]-s-triazine.

Examples of the α,β-unsaturated double bond group-containing compound having both a nitrogen atom and an oxygen atom as hetero atoms on the cyclic structure include a (meth)acrylate ester compound having a cyclic structure containing an oxygen atom in addition to a nitrogen atom, such as imide (meth)acrylate, 2-(4-oxazolin-3-yl)ethyl (meth)acrylate, ethoxylated isocyanuric acid tri(meth)acrylate, and ε-caprolactone-modified tris(2-acryloyloxyethyl) isocyanurate; a (meth)acrylamide compound having a cyclic structure, such as 4-acryloylmorpholine; an N-vinyl group-containing compound having a cyclic structure having both a nitrogen atom and an oxygen atom, such as N-vinyl-2-pyrrolidone and N-vinyl-ε-caprolactam; a maleimide derivative compound having both a nitrogen atom and an oxygen atom, such as maleimide, methylmaleimide, ethylmaleimide, propylmaleimide, butylmaleimide, octylmaleimide, dodecylmaleimide, stearylmaleimide, phenylmaleimide, and cyclohexylmaleimide; and an ethenyl group-containing compound having an oxygen atom in addition to a nitrogen atom, such as 2-vinyloxazole, 2-phenyl-4-vinyloxazole, 2-phenyl-5-vinyloxazole, 5-ethoxy-2-vinyloxazole, 3-vinyl-5-nitrosooxazole, 2-vinyl-4,5-diphenyloxazole, 2-vinyl-2-oxazoline, 4,4-dimethyl-2-vinyl-2-oxazolin-5-one, 2-vinylbenzoxazole, and 1-vinylpyridin-2(1H)-one.

Examples of the α,β-unsaturated double bond group-containing compound having both a nitrogen atom and a sulfur atom as hetero atoms on the cyclic structure include a vinyl group-containing compound having a cyclic structure containing a sulfur atom in addition to a nitrogen atom, and specific examples thereof include 2-vinylthiazole, 4-methyl-5-vinylthiazole, 2-vinylbenzothiazole, 2-[2-(1-naphthyl)vinyl]benzothiazole, 2-[2-(dimethylamino)vinyl]benzothiazole, and 1-vinyl-2(1H)-pyridinethione.

From the standpoint of imparting the excellent adhesiveness to a tooth substance to the dental composition, the dental composition preferably contains a monomer having an acidic group in some embodiments (such as a dental self-adhesive composite resin and dental cement).

A monomer having an acidic group contained in the dental composition can enhance the adhesion strength to a tooth substance.

The monomer having an acidic group used is preferably a radical polymerizable monomer. Specific examples of the radical polymerizable monomer used as the monomer having an acidic group include a (meth)acrylate based monomer, a (meth)acrylamide based monomer, an ester compound of α-cyanoacrylic acid, (meth)acrylic acid, an α-halogenated acrylic acid, crotonic acid, cinnamic acid, sorbic acid, maleic acid, itaconic acid, and the like, a vinyl ester compound, a vinyl ether compound, a mono-N-vinyl derivative, and a styrene derivative. Among these, a (meth)acrylate based monomer and a (meth)acrylamide based monomer are preferred from the standpoint of the curability.

Examples of the monomer having an acidic group include a monomer having at least one acidic group, and examples thereof include a phosphoric acid group, a pyrophosphoric acid group, a thiophosphoric acid group, a phosphonic acid group, a carboxylic acid group, and a sulfonic acid group.

One kind of the monomer having an acidic group may be used alone, or two or more kinds thereof may be used appropriately in combination. Specific examples of the monomer having an acidic group are described below.

Examples of the monomer having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl-2-dihydrogen phosphate, 2-(meth)acryloyloxyethyl phenyl hydrogen phosphate, 2-(meth)acryloyloxyethyl-(2-bromoethyl) hydrogen phosphate, 2-methacryloyloxyethyl-(4-methoxyphenyl) hydrogen phosphate, 2-methacryloyloxypropyl-(4-methoxyphenyl) hydrogen phosphate, and oxychlorides, alkali metal salts, and amine salts thereof, in which a monomer having a divalent phosphoric acid group having an alkylene group having 6 to 12 carbon atom is preferred.

Examples of one preferred embodiment of the dental composition of the present invention include a self-adhesive dental composite resin containing a monomer having an acidic group containing a monomer having a divalent phosphoric acid group having an alkylene group having 6 to 12 carbon atom.

Examples of the monomer having a pyrophosphoric acid group include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, and oxychlorides, alkali metal salts, and amine salts thereof.

Examples of the monomer having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyeicosyl dihydrogen thiophosphate, and oxychlorides, alkali metal salts, and amine salts thereof.

Examples of the monomer having a phosphonic acid group include 2-(meth)acryloyloxyethyl phenylphosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and oxychlorides, alkali metal salts, and amine salts thereof.

Examples of the monomer having a carboxylic acid group include a (meth)acrylate ester having one carboxy group or an acid anhydride group thereof in one molecule and a (meth)acrylate ester having multiple carboxy groups or an acid anhydride group thereof in one molecule.

Examples of the monomer having one carboxy group or an acid anhydride group thereof in one molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen maleate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds obtained by converting the carboxy group of these compounds to an acid anhydride group.

Examples of the monomer having multiple carboxy groups or acid anhydride groups thereof in one molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethyl naphthalene-1,2,6-tricarboxylic anhydride, 6-(meth)acryloyloxyethyl naphthalene-2,3,6-tricarboxylic anhydride, 4-(meth)acryloyloxyethylcarbonylpropionoyl-1,8-naphthalic anhydride, and 4-(meth)acryloyloxyethyl naphthalene-1,8-tricarboxylic anhydride.

Examples of the monomer having a sulfonic acid group include 2-sulfoethyl (meth)acrylate.

Among the monomers having an acidic group described above, from the standpoint of achieving a good adhesion strength in the case of using as a self-adhesive dental composite resin or dental cement, the dental composition preferably contains a monomer having a phosphoric acid group or a monomer having a carboxylic acid group, in which 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-methacryloyloxyethyl dihydrogen phosphate and bis(2-methacryloyloxyethyl) hydrogen phosphate are more preferred, and 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyeicosyl dihydrogen phosphate are more preferred, and from the standpoint of the balance with the curability, 10-(meth)acryloyloxydecyl dihydrogen phosphate is particularly preferred.

In one embodiment, the content of the monomer having an acidic group in the dental composition is preferably 0.1% by mass of more, more preferably 0.2% by mass or more, further preferably 0.5% by mass or more, and particularly preferably 1% by mass or more, based on 100% by mass in total of the dental composition, from the standpoint of the adhesiveness to a tooth substance.

The content thereof is preferably 25% by mass or less, more preferably 20% by mass or less, further preferably 15% by mass or less, and particularly preferably 10% by mass or less, based on 100% by mass in total of the dental composition, from the standpoint of the adhesiveness to a tooth substance.

### (Contents of Monomer (A) and Monomers (A-1) and (A-2))

The total mass of the monomer (A-1) and the monomer (A-2) (i.e., the mass of the monomer (A)) in the dental composition is preferably 10% by mass or more and less than 50% by mass, more preferably 15 to 45% by mass, further preferably 20 to 42% by mass, and still further preferably 25 to 40% by mass, based on 100% by mass in total of the dental composition.

The dental composition preferably contains the monomer (A-1) in an amount of 1 to 80 parts by mass, more preferably 5 to 75 parts by mass, further preferably 10 to 70 parts by mass, still further preferably 15 to 65 parts by mass, still more further preferably 18 to 60 parts by mass, even further preferably 18 to 53 parts by mass, even still further preferably 18 to 51 parts by mass, even still more further preferably 18 to 50 parts by mass, and particularly preferably 20 to 50 parts by mass, per 100 parts by mass in total of the monomer (A-1) and the monomer (A-2).

In the case where the content of the monomer (A-1) in the dental composition is in the range, the dental composition can readily has a high depth of photocuring, and a cured product thereof readily has a high mechanical strength, a low polymerization shrinkage stress, and a high surface hardness.

The dental composition preferably contains the monomer (A-2) in an amount of 20 to 99 parts by mass, more preferably 25 to 95 parts by mass, further preferably 30 to 90 parts by mass, still further preferably 35 to 85 parts by mass, still more further preferably 40 to 82 parts by mass, even further preferably 47 to 82 parts by mass, even still further preferably 49 to 82 parts by mass, even still more further preferably 50 to 82 parts by mass, and particularly preferably 50 to 80 parts by mass, per 100 parts by mass in total of the monomer (A-1) and the monomer (A-2).

In the case where the content of the monomer (A-2) in the dental composition is in the range, the dental composition readily has a high depth of photocuring, and a cured product thereof readily has a high mechanical strength.

In the dental composition, the ratio M1/M2 of the mass M1 of the monomer (A-1) and the mass M2 of the monomer (A-2) is preferably 5/95 to 75/25, more preferably 10/90 to 70/30, further preferably 15/85 to 65/35, still further preferably 18/82 to 60/40, still more further preferably 18/82 to 53/47, even further preferably 18/82 to 51/49, even still further preferably 18/82 to 50/50, and particularly preferably 20/80 to 50/50, from the aforementioned standpoint.

### <Polymerization Initiator (B)>

The polymerization initiator (B) used may be selected from polymerization initiators that are used in the general industrial fields, in which polymerization initiators that are used in the dental field are preferably used. Among them, a photopolymerization initiator is particularly preferred.

One kind of the polymerization initiator (B) may be used alone, or two or more kinds thereof may be used in combination.

Preferred examples of the photopolymerization initiator include a (bis)acylphosphine oxide compound, a ketal compound, an α-diketone compound, and a coumarin compound.

Examples of an acylphosphine oxide compound in the (bis)acylphosphine oxide compound include 2,4,6-trimethylbenzoyl diphenylphosphine oxide, 2,6-dimethoxybenzoyl diphenylphosphine oxide, 2,6-dichlorobenzoyl diphenylphosphine oxide, 2,4,6-trimethylbenzoyl methoxyphenylphosphine oxide, 2,4,6-trimethylbenzoyl ethoxyphenylphosphine oxide, 2,3,5,6-tetramethylbenzoyl diphenylphosphine oxide, benzoyl di(2,6-dimethylphenyl) phosphonate, and salts (such as a sodium salt, a potassium salt, and an ammonium salt) thereof.

Examples of a bisacylphosphine oxide compound in the (bis)acylphosphine oxide compound include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts (such as a sodium salt, a potassium salt, and an ammonium salt) thereof, and also include sodium phenyl(2,4,6-trimethylbenzoyl)phosphinate, lithium phenyl(2,4,6-trimethylbenzoyl)phosphinate, sodium bis(2,4,6-trimethylbenzoyl)phosphinate, and lithium bis(2,4,6-trimethylbenzoyl)phosphinate.

Among the (bis)acylphosphine oxide compounds, 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and a sodium salt of 2,4,6-trimethylbenzoylphenylphosphine oxide are particularly preferred. In one embodiment (such as a dental self-adhesive composite resin and dental cement), sodium bis(2,4,6-trimethylbenzoyl)phosphinate and lithium bis(2,4,6-trimethylbenzoyl)phosphinate are preferred from the standpoint of the adhesiveness to a tooth substance.

Examples of the ketal compound include benzyl dimethyl ketal and benzyl diethyl ketal.

Examples of the α-diketone compound include diacetyl, benzil, camphorquinone, 2,3-petanedione, 2,3-octanedione, 9,10-phenanthrenedione, 4,4'-oxybenzil, and acenaphthoquinone. Among these, camphorquinone is preferred from the standpoint of the maximum absorption wavelength thereof in the visible region.

Examples of the coumarin compound include the compounds described in JPH 9-3109 A and JPH 10-245525 A, and specific examples thereof include 3,3'-carbonylbis(7-diethylaminocoumarin), 3-(4-methoxybenzoyl)coumarin, 3-thienylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazoyl)-7-(diethylamino)coumarin, 3-(2-benzothiazoyl)-7-(dibutylamino)coumarin, 3-(2-benzimidazoyl)-7-(diethylamino)coumarin, 3-(2-benzothiazoyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrano[6,7,8-ij]quinolizin-11-one, and 10-(2-benzothiazoyl)-2,3,6,7-tetrahydro-1,1,7,7-tetramethyl-1H,5H,11H-[1]benzopyrano[6,7,8-ij]quinolizin-11-one.

Among the coumarin compounds described above, 3,3'-carbonylbis(7-diethylaminocoumarin) and 3,3'-carbonylbis(7-dibutylaminocoumarin) are particularly preferred.

Among the photopolymerization initiators described above, the (bis)acylphosphine oxide compound, the α-diketone compound, and the coumarin compound are excellent in ability to initiate photopolymerization in the visible region and the near ultraviolet region, and therefore can initiate the polymerization by using a light source (such as a halogen lamp, a light-emitting diode (LED), and a xenon lamp).

The dental composition may further contain a chemical polymerization initiator from the standpoint of enabling chemical polymerization in one embodiment (particularly dental cement and a dental core build-up material), and an organic peroxide is preferably used therefor.

The organic peroxide used as the chemical polymerization initiator is not particularly limited, and a known compound can be used. Examples of the representative organic peroxide include a ketone peroxide, a hydroperoxide, a diacyl peroxide, a dialkyl peroxide, a peroxyketal, a peroxyester, and a peroxydicarbonate. Specific examples of the organic peroxide include the compounds described in WO 2008/087977. One kind of the chemical polymerization initiator may be used alone, or two or more kinds thereof may be used in combination. The chemical polymerization initiator allows redox polymerization to occur by combining with a polymerization accelerator for chemical polymerization, and causes curing. The polymerization accelerator to be combined is not particularly limited, and examples thereof include an amine compound, a sulfinic acid and a salt thereof, a benzotriazole compound, a benzimidazole compound, a reducing inorganic compound having sulfur, and a thiourea compound. One kind of the polymerization accelerator may be used alone, or two or more kinds thereof may be used in combination. The chemical polymerization initiator and the polymerization accelerator for chemical polymerization are preferably packaged separately from each other from the standpoint of the occurrence of chemical polymerization, and for example, a two-component paste is preferred.

The amount of the polymerization initiator (B) used is not particularly limited, and is preferably 0.1 to 20 parts by mass, more preferably 0.2 to 15 parts by mass, and further preferably 0.3 to 10 parts by mass, per 100 parts by mass in total of the monomer (A) in the dental composition, from the standpoint of favorably facilitating the cure of the dental composition.

### <Filler (C)>

Any filler can be used as the filler (C) unless the effects of the present invention are impaired, and examples thereof include an inorganic filler, an organic filler, and a composite filler of an inorganic filler and an organic filler. One kind of the filler (C) may be blended alone, or two or more kinds thereof may be blended in combination.

The average particle diameter of the filler (C) is preferably 0.001 to 10 µm, more preferably 0.002 to 5 µm, further preferably 0.003 to 3 µm, still further preferably 0.004 to 1 µm, and still more further preferably 0.005 to 0.8 µm. The average particle diameter of the filler (C) means an average primary particle diameter obtained through the laser diffraction-scattering method or the observation of the particles with an electron microscope as described later.

Examples of the inorganic filler include amorphous inorganic particles and inorganic ultrafine particles.

Examples of the amorphous inorganic particles include various kinds of glass (which contains silica as the major component, and contains an oxide of a heavy metal, boron, aluminum, and the like depending on necessity, for example, dental glass powder, such as E-glass, barium glass ("GM27884" and "GM8235", product names, available from Schott AG, and "E2000" and "E3000", product names, available from Esstech, Inc.), and lanthanum glass ceramics ("GM31684", a product name, available from Schott AG)), various kinds of ceramics, a composite oxide, such as silica-titania and silica-zirconia, kaolin, a clay mineral (such as montmorillonite), mica, ytterbium fluoride, silica-coated ytterbium fluoride ("SG-YBF100WSCMP10", a product name, available from Sukgyung AT Co., Ltd.), and yttrium fluoride.

Examples of the inorganic ultrafine particles include inorganic oxide particles, such as silica, alumina, titania, and zirconia, or composite oxide particles containing them, and particles of calcium phosphate, hydroxyapatite, yttrium fluoride, ytterbium fluoride, barium titanate, and potassium titanate. Preferred examples thereof include particles of silica, alumina, titania, a silica-alumina composite oxide, and a silica-zirconia composite oxide, which are produced by a flame pyrolysis, and examples thereof include "Aerosil (registered trademark) 50", "Aerosil (registered trademark) 130", "Aerosil (registered trademark) 380", "Aerosil (registered trademark) OX50", "Aeroxide (registered trademark) AluC", "Aeroxide (registered trademark) TiO₂ P25", "VP Zirconium Oxide 3-YSZ", and "VP Zirconium Oxide3-YSZPH", product names, available from Nippon Aerosil Co., Ltd.).

In the description herein, in the case where the inorganic filler is surface-treated as described later, the average particle diameter of the inorganic filler means the average particle diameter before the surface treatment.

The inorganic ultrafine particles may be used in the form of agglomerated particles formed through agglomeration of inorganic ultrafine particles. In general, commercially available inorganic ultrafine particles exist in the form of agglomerated material, but have weak agglomeration power in such an extent that the agglomerated particles are dispersed to the particle diameter provided by the manufacturer in such a manner that 10 mg of the inorganic ultrafine particle powder is added to 300 mL of water or water having 5% by mass or less of a surfactant, such as sodium hexametaphosphate, added thereto (dispersion medium), and dispersed with ultrasonic wave having an output power of 40 W and a frequency of 39 kHz for 30 minutes. However, the agglomerated particles used as the filler (C) may be particles that are firmly agglomerated to such an extent that the agglomerated particles are substantially not dispersed under the aforementioned condition.

As a method for producing the firmly agglomerated particles from commercially available inorganic ultrafine particles, by further enhancing the agglomeration power, it is preferred to use a method of heating the inorganic ultrafine particles to a temperature immediately before the particles melt, so as to heat the inorganic ultrafine particles to such an extent that the particles in contact with each other are slightly fused to each other. In this case, for controlling the shape of the agglomerated particles, the agglomerated form may be produced before heating. Examples of the method therefor include a method of pressurizing the inorganic ultrafine particles placed in a suitable vessel, and a method of once dispersing the particles in a solvent, and then removing the solvent by such a method as spray drying.

As another preferred method for producing the firmly agglomerated inorganic ultrafine particles, agglomerated particles including particles that are firmly agglomerated to each other can be readily obtained in such a manner that silica sol, alumina sol, titania sol, zirconia sol, or the like produced by a wet method are dried by such a method as freeze drying or spray drying, and then subjected to a heat treatment depending on necessity. Specific examples of the sol include "Seahostar", a product name, available from Nippon Shokubai Co., Ltd., "Oscal" and "Queen Titanic", product names, available from JGC Catalysts and Chemicals Ltd., and "Snowtex", "Alumina Sol", "Celnax", "NanoUse", product names, available from Nissan Chemical Corporation. The shape of the inorganic ultrafine particles is not particularly limited, and can be appropriately selected. Commercially available aggregated particles of inorganic ultrafine particles may be directly used, and examples thereof include "Silica Microbead P-500" and "Silica Microbead P-1500", product names, available from JGC Catalysts and Chemicals Ltd., and silica-zirconia ("SG-SZ200G151CMP8", a product name, available from Sukgyung AT Co., Ltd.).

Examples of the organic filler include polymethyl methacrylate, polyethyl methacrylate, a polymer of a polyfunctional methacrylate, polyamide, polystyrene, polyvinyl chloride, chloroprene rubber, nitrile rubber, and styrene-butadiene rubber.

Examples of the composite filler of an inorganic filler and an organic filler include a filler obtained by dispersing an inorganic filler in an organic filler and an inorganic-organic composite filler obtained by coating an inorganic filler with various polymers.

The filler may be surface-treated before use, with a known surface treatment agent (such as a silane coupling agent) for enhancing the curability, the mechanical strength, and the handleability. Examples of the surface treatment agent include methacryloyloxymethyltrimethoxysilane, 2-methacryloyloxyethyltrimethoxysilane, 3-methacryloyloxypropyltrimethoxysilane, 4-methacryloyloxybutyltrimethoxysilane, 5-methacryloyloxypentyltrimethoxysilane, 6-methacryloyloxyhexyltrimethoxysilane, - methacryloxyoctyltrimethoxysilane, 9-methacryloyloxynonyltrimethoxysilane, 10-methacryloyloxydecyltrimethoxysilane, 11-methacryloyloxyundecyltrimethoxysilane, 11-methacryloyloxyundecyldichloromethylsilane, 11-methacryloyloxyundecyltrichlorosilane, 11-methacryloyloxyundecyldimethoxymethylsilane, 12-methacryloyloxydodecyltrimethoxysilane, and 13-methacryloyloxytridecyltrimethoxysilane.

The average particle diameter (average primary particle diameter) can be obtained through the laser diffraction-scattering method or the observation with an electron microscope. Specifically, the laser diffraction-scattering method is convenient for measuring the particle diameter of the particles having an average particle diameter of 0.1 µm or more, and the observation with an electron microscope is convenient for measuring the particle diameter of the particles having an average particle diameter of less than 0.1 µm. The laser diffraction-scattering method may be performed, for example, by measuring on volume basis with a laser diffraction particle diameter distribution analyzer (SALD-2300, available from Shimadzu Corporation) using a 0.2% sodium hexametaphosphate aqueous solution as a dispersion medium. The observation with an electron microscope may be performed by using a scanning electron microscope (for example, SU-3800 and S-4000, available from Hitachi High-Technologies Corporation). In the observation with an electron microscope, the measurement may be performed in such a manner that an electron micrograph of the particles is obtained, and the particles (200 or more) observed in a unit view field in the micrograph are measured with an image analyzing particle size distribution analysis software (Mac-View (available from Mountech Co., Ltd.)). At this time, the particle diameter is obtained as an arithmetic average value of the longest diameter and the shortest diameter of the particle, and the average primary particle diameter is calculated from the number of the particles and the particle diameter.

The content of the filler (C) is 50% by mass to 90% by mass based on 100% by mass in total of the dental composition as described above, and is preferably 55 to 85% by mass, more preferably 58 to 80% by mass, and further preferably 60 to 75% by mass, from the standpoint of the handleability of the paste and the mechanical strength of the cured product.

The content of the filler (C) is preferably 50 to 1,000 parts by mass, more preferably 100 to 500 parts by mass, and further preferably 100 to 300 parts by mass, per 100 parts by mass in total of the monomer (A) in the dental composition, from the standpoint of the handleability of the paste and the mechanical strength of the cured product. Within the ranges, the cured product that is good in all the mechanical strength and the surface hardness can be obtained.

### <Polymerization Accelerator (D)>

The dental composition according to an embodiment of the present invention may contain a polymerization accelerator (D).

Examples of the polymerization accelerator (D) include an amine compound, a sulfinic acid compound (including a salt), a barbituric acid derivative, a triazine compound, a copper compound, a tin compound, a vanadium compound, a halogen compound, an aldehyde compound, a thiol compound, a sulfite salt, a hydrogen sulfite salt, and a thiourea compound.

One kind of the polymerization accelerator (D) may be used alone, or two or more kinds thereof may be used in combination.

The amine compound is classified into an aliphatic amine and an aromatic amine.

Examples of the aliphatic amine include a primary aliphatic amine, a secondary aliphatic amine, and a tertiary aliphatic amine.

Examples of the primary aliphatic amine include n-butylamine, n-hexylamine, and n-octylamine.

Examples of the secondary aliphatic amine include diisopropylamine, dibutylamine, and N-methylethanolamine.

Examples of the tertiary aliphatic amine include N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine.

Among these, a tertiary aliphatic amine is preferred, and N-methyldiethanolamine and triethanolamine are more preferred, from the standpoint of the curability and the storage stability of the dental composition.

Examples of the aromatic amine include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-tert-butylaniline, N,N-bis(2-hydroxyethyl)-3,5-diisopropylaniline, N,N-bis(2-hydroxyethyl)-3,5-di-tert-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-tert-butylaniline, N,N-dimethyl-3,5-di-tert-butylaniline, ethyl 4-(N,N-dimethylamino)benzoate, methyl 4-(N,N-dimethylamino)benzoate, 2-butoxyethyl 4-(N,N-dimethylamino)benzoate, 2-((meth)acryloyloxy)ethyl 4-(N,N-dimethylamino)benzoate, 4-(N,N-dimethylamino)benzophenone, and butyl 4-(N,N-dimethylamino)benzoate.

Among these, N,N-bis(2-hydroxyethyl)-p-toluidine, ethyl 4-(N,N-dimethylamino)benzoate, 2-butoxyethyl 4-(N,N-dimethylamino)benzoate, and 4-(N,N-dimethylamino)benzophenone are preferred from the standpoint of imparting the excellent curability to the dental composition.

Examples of the sulfinic acid compound include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate.

Among these, sodium benzenesulfinate, sodium p-toluenesulfinate, and sodium 2,4,6-triisopropylbenzenesulfinate are particularly preferred.

Examples of the barbituric acid derivative include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, a thiobarbituric acid compound, and salts thereof.

Examples of a salt of these barbituric acid derivative include an alkali metal salt and an alkaline earth metal salt, and specific examples thereof include sodium 5-butylbarbiturate, sodium 1,3,5-trimethylbarbiturate, and sodium 1-cyclohexyl-5-ethylbarbiturate.

The barbituric acid derivative is particularly preferably 5-butylbarbituric acid, 1,3,5-trimethylbarbituric acid, 1-cyclohexyl-5-ethylbarbituric acid, 1-benzyl-5-phenylbarbituric acid, and sodium salts thereof.

Examples of the triazine compound include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N,N-bis(2-hydroxyethyl)amino}ethoxy]-4,6-bis(trichloromethyl)-s-triazine, 2-[2-{N-hydroxyethyl-N-ethylamino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

Among these triazine compounds, 2,4,6-tris(trichloromethyl)-s-triazine is preferred from the standpoint of the polymerization activity, and 2-phenyl-4,6-bis(trichloromethyl)-s-triazine, 2-(p-chlorophenyl)-4,6-bis(trichloromethyl)-s-triazine, and 2-(4-biphenylyl)-4,6-bis(trichloromethyl)-s-triazine are preferred from the standpoint of the storage stability. One kind of the triazine compound may be used alone, or two or more kinds thereof may be used in combination.

Examples of the copper compound include copper acetylacetonate, copper(II) acetate, copper oleate, copper(II) chloride, and copper(II) bromide.

Examples of the tin compound include di-n-butyltin dimaleate, di-n-octyltin dimaleate, di-n-octyltin dilaurate, and di-n-butyltin dilaurate. Among these, di-n-octyltin dilaurate and di-n-butyltin dilaurate are preferred.

The vanadium compound is preferably a tetravalent or pentavalent vanadium compound. Examples of the tetravalent or pentavalent vanadium compound include divanadium(IV) tetraoxide, vanadium(IV) oxide acetylacetonate, vanadyl oxalate (IV), vanadyl sulfate (IV), oxobis(1-phenyl-1,3-butanedionato)vanadium(IV), bis(maltolato) oxovanadium(IV), vanadium(V) pentoxide, sodium metavanadate (V), and ammonium metavanadate (V).

Examples of the halogen compound include dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the aldehyde compound include terephthalaldehyde and a benzaldehyde derivative. Examples of the benzaldehyde derivative include dimethylaminobenzaldehyde, p-methoxybenzaldehyde, p-ethoxybenzaldehyde, and p-n-octyloxybenzaldehyde. Among these, p-n-octyloxybenzaldehyde is preferred from the standpoint of the curability.

Examples of the thiol compound include 3-mercaptopropyltrimethoxysilane, 2-mercaptobenzoxazole, decanethiol, and thiobenzoic acid.

Examples of the sulfite salt include sodium sulfite, potassium sulfite, calcium sulfite, and ammonium sulfite.

Examples of the hydrogen sulfite salt include sodium hydrogen sulfite and potassium hydrogen sulfite.

Examples of the thiourea compound include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, and tetracyclohexylthiourea.

The content of the polymerization accelerator (D) in the dental composition is not particularly limited, and is preferably 0.1 to 20 parts by mass, more preferably 0.2 to 15 parts by mass, and further preferably 0.2 to 10 parts by mass, per 100 parts by mass in total of the monomer (A) in the dental composition, from the standpoint of the curability of the resulting dental composition, and the like.

### <Additional Component>

The dental composition according to an embodiment of the present invention may contain a known additive in such a range that does not cause the capabilities to fall below the practically usable level. Examples of the additive include a polymerization inhibitor, an antioxidant, a colorant (such as a pigment and a dye), an ultraviolet ray absorbent, a solvent (such as water and an organic solvent), and a thickener. One kind of the additive may be used alone, or two or more kinds thereof may be used in combination.

In one embodiment, the content of a solvent (such as water and an organic solvent) in the dental composition is preferably less than 1% by mass, more preferably less than 0.1% by mass, and further preferably less than 0.01% by mass, based on the total amount of the dental composition.

Examples of the polymerization inhibitor include hydroquinone, hydroquinone monomethyl ether, dibutylhydroquinone, dibutylhydroquinone monomethyl ether, tert-butylcatechol, 2-tert-butyl-4,6-dimethylphenol, 2,6-di-tert-butylphenol, and 3,5-di-tert-butyl-4-hydroxytoluene.

The content of the polymerization inhibitor is preferably 0.001 to 1.0 part by mass per 100 parts by mass in total of the monomers in the dental composition.

The total mass of the additional additives in the dental composition is not particularly limited, as long as exhibiting the effects of the present invention, and is preferably 20% by mass or less, more preferably 15% by mass or less, further preferably 10% by mass or less, still further preferably 5% by mass or less, and still more further preferably 2% by mass or less, and is 0% by mass or more, based on 100% by mass of the dental composition, from the standpoint of further facilitating the effects of the present invention. In other words, the mass of the additional additives in the dental composition is preferably 0 to 20% by mass based on 100% by mass of the dental composition.

### <Total Content of Components (A) to (C)>

The total mass of the monomer (A), the polymerization initiator (B), and the filler (C) in the dental composition is preferably 90% by mass or more, more preferably 95% by mass or more, further preferably 98% by mass or more, and still further preferably 99% by mass or more, and may be 100% by mass, from the standpoint of facilitating the securement of the prescribed depth of photocuring, and facilitating the achievement of the prescribed polymerization shrinkage stress and mechanical strength by the cured product thereof. In other words, the total mass of the monomer (A), the polymerization initiator (B), and the filler (C) in the dental composition is preferably 90 to 100% by mass.

### [Production Method of Dental Composition]

In producing the dental composition, for example, the addition order and the mixing method of the components are not particularly limited, and the dental composition can be produced by a method that is known by a skilled person in the art. Preferred examples thereof include a method of mixing the components other than the filler (C), and the adding and mixing the filler (C).

### [Application of Dental Composition]

In one embodiment, the dental composition is a dental composite resin.

In another embodiment the dental composition is dental cement.

In still another embodiment, the dental composition is a self-adhesive dental composite resin.

In still more another embodiment, the dental composition is a dental core build-up material.

The dental composition may be a one-component type or a two-component type. In the two-component type, for example, for enhancing the storage stability, particular multiple components may be separately packaged in the first package and the second package.

### <Properties of Dental Composition>

### (Polymerization Shrinkage Stress)

The dental composition preferably has a small polymerization shrinkage stress from the standpoint of facilitating the achievement of the prescribed shape. In more detail, in the case where the dental composition is charged in a stainless steel washer having an inner diameter of 5.3 mm and a thickness of 0.8 mm, and cured through light irradiation, the polymerization shrinkage stress occurring therein measured with a universal tester is preferably 12.0 MPa or less, more preferably 9.7 MPa or less, further preferably 9.5 MPa or less, still further preferably 8.5 MPa or less, still more further preferably 8.0 MPa or less, and particularly preferably 7.5 MPa or less. The lower limit thereof is not particularly limited, and for example, may be 3.5 MPa or more from the standpoint of the mechanical strength of the cured product. In other words, the polymerization shrinkage stress of the dental composition is preferably 3.5 to 10.0 MPa.

The polymerization shrinkage stress can be measured by the method described in detail in the examples described later.

The polymerization shrinkage stress can be set to the aforementioned range, for example, by selecting and regulating at least one item selected from the group consisting of the kind of the monomer (A), the amount of the monomer (A) blended, the kind of the polymerization initiator (B), the amount of the polymerization initiator (B) blended, the kind of the filler (C), and the amount of the filler (C) blended.

### (Flexural Modulus and Flexural Strength)

The cured product of the dental composition preferably has a high flexural modulus and a high flexural strength for sufficiently withstanding an occlusal load in chewing hard foods and the like.

The flexural modulus of the dental composition is a value that is calculated from the result of the flexural strength test measured according to ISO 4049:2019, and is preferably 4.0 GPa or more, more preferably 5.0 GPa or more, further preferably 7.0 GPa or more, still further preferably 7.5 GPa or more, and still more further preferably 8.0 GPa or more. The upper limit thereof is not particularly limited, and for example, may be 20 GPa or less from the standpoint of the manufacturability. In other words, the flexural modulus of the cured product of the dental composition is preferably 4.0 to 20 GPa.

The flexural strength of the dental composition is a value that is measured according to ISO 4049:2019, and is preferably 100 MPa or more, more preferably 110 MPa or more, further preferably 120 MPa or more, still further preferably 125 MPa or more, and still more further preferably 130 MPa or more. The upper limit thereof is not particularly limited, and for example, may be 200 MPa or less from the standpoint of the handleability of the paste, and the like. In other words, the flexural strength of the cured product of the dental composition is preferably 100 to 200 MPa.

The flexural modulus and the flexural strength can be measured by the method described in detail in the examples described later.

The flexural modulus and the flexural strength can be set to the aforementioned ranges, for example, by selecting and regulating at least one item selected from the group consisting of the kind of the monomer (A), the amount of the monomer (A) blended, the kind of the polymerization initiator (B), the amount of the polymerization initiator (B) blended, the kind of the filler (C), and the amount of the filler (C) blended.

### (Vickers Hardness)

The cured product of the dental composition preferably has a high surface hardness for facilitating the prevention of damage due to contact with foreign matters. In more detail, the surface of the cured product obtained through light irradiation of the dental composition is mirror polished, the value of Vickers hardness of the obtained surface measured with a micro hardness testing machine is preferably 30 Hv or more, more preferably 35 Hv or more, further preferably 40 Hv or more, and still further preferably 45 Hv or more. The upper limit thereof is not particularly limited, and for example, may be 100 Hv or less from the standpoint of the handleability of the paste, and the like. In other words, the Vickers hardness of the dental composition is preferably 30 to 100 Hv.

The Vickers hardness can be measured by the method described in detail in the examples described later.

The Vickers hardness can be set to the aforementioned range, for example, by selecting and regulating at least one item selected from the group consisting of the kind of the monomer (A), the amount of the monomer (A) blended, the kind of the polymerization initiator (B), the amount of the polymerization initiator (B) blended, the kind of the filler (C), and the amount of the filler (C) blended.

### (Depth of Photocuring)

The dental composition preferably has a large depth of photocuring from the standpoint of facilitating the progress of curing. In more detail, the value that is measured according to ISO 4049:2019 is preferably 3.0 mm or more, more preferably 3.5 mm or more, further preferably 4.0 mm or more, still further preferably 4.5 mm or more, still more further preferably 5.0 mm or more, and particularly preferably 5.5 mm or more. The upper limit thereof is not particularly limited, and for example, may be 5.9 mm or less from the standpoint of the handleability of the paste, and the like. In other words, the depth of photocuring of the dental composition is preferably 3.0 to 5.9 mm.

The depth of photocuring can be measured by the method described in detail in the examples described later.

The depth of photocuring can be set to the aforementioned range, for example, by selecting and regulating at least one item selected from the group consisting of the kind of the monomer (A), the amount of the monomer (A) blended, the kind of the polymerization initiator (B), the amount of the polymerization initiator (B) blended, the kind of the filler (C), and the amount of the filler (C) blended.

### Examples

The present invention is described more specifically with reference to examples, but the present invention is not limited to the examples.

The components used in Examples and Comparative Examples are shown below along with the symbols therefor.

### [Monomer (A)]

### <Monomer (A-1)>

1-NMMA: 1-naphthylmethyl methacrylate
1-NMA: 1-naphthylmethyl acrylate

### <Monomer (A-2)>

UDMA: 2,2,4-trimethylhexamethylene bis(2-carbamoyloxyethyl) dimethacrylate (available from Kyoeisha Chemical Co., Ltd.)
POB-MA: m-phenoxybenzyl methacrylate (available from Kyoeisha Chemical Co., Ltd.)
D-2.6E: 2,2-bis(4-(meth)acryloyloxypolyethoxyphenyl)propane
3G: triethylene glycol dimethacrylate
THF-MA: tetrahydrofurfuryl methacrylate
MDP: 10-(meth)acryloyloxydecyl dihydrogen phosphate

### [Polymerization Initiator (B)]

CQ: camphorquinone
TMDPO: 2,4,6-trimethylbenzoyl diphenylphosphine oxide

### [Filler (C)]

Filler 1: surface-treated SiO₂ treated silica produced by the procedure below
Filler 2: surface-treated BaO filler produced by the procedure below
Filler 3: commercially available surface-treated SiO₂-ZrO₂ agglomerated filler
Filler 4: commercially available surface-treated SiO₂-coated YbF₃

### [Polymerization Accelerator (D)]

DABE: ethyl 4-(N,N-dimethylamino)benzoate

### [Additive]

BHT: 3,5-di-tert-butyl-4-hydroxytoluene

### [Preparation of Filler]

### <Filler 1>

In a three-neck flask, 100 g of Ar130 (hydrophilic fumed silica, silica ultrafine particles "Aerosil (registered trademark) 130", average particle diameter: 16 nm, refractive index: 1.46, available from Nippon Aerosil Co., Ltd.), 30 g of 3-methacryloyloxypropyltrimethoxysilane, and 200 mL of a 0.3% by mass acetic acid aqueous solution were placed, and agitated at room temperature under ultrasonic dispersion for 2 hours. After removing water through freeze drying, the mixture was heated to 90°C for 3 hours to produce surface-treated SiO₂ treated silica, which was used as a filler 1.

### <Filler 2>

In a three-neck flask, 100 part by mass of barium glass ("GM27884", a product name, average particle diameter of primary particles: 0.18 µm, available from Schott AG), 11 parts by mass of 3-methacryloyloxypropyltrimethoxysilane, and 500 mL of a 0.5% by mass acetic acid aqueous solution were placed and agitated at room temperature under ultrasonic dispersion for 2 hours. After removing water through freeze drying, the mixture was heated to 90°C for 3 hours to produce a surface-treated BaO filler, which was used as a filler 2.

### <Filler 3>

A commercially available surface-treated SiO₂-ZrO₂ agglomerated filler ("SG-SZ200G151CMP8", a product name, average particle diameter of primary particles: 200 nm, average particle diameter of secondary particles: 5.2 µm, refractive index: 1.51, available from Sukgyung AT Co., Ltd.) was used as a filler 3.

### <Filler 4>

A commercially available SiO₂-coated YbF₃ ("SG-YBF100WSCMP10", a product name, average particle diameter of primary particles: 110 nm, average particle diameter of secondary particles: 1.2 µm, refractive index: 1.53, available from Sukgyung AT Co., Ltd.) was used as a filler 4.

### [Examples 1 to 11 and Comparative Examples 1 to 6]

Among the components shown in Table 1 below, the components in the masses shown in the columns except for the column "Filler" were mixed at ordinary temperature (25°C), and then the component in the mass shown in the column "Filler" was added thereto and mixed therewith, so as to prepare dental compositions of Examples 1 to 11 and Comparative Examples 1 to 5. All the compositions were in the form of paste, and had fluidity that was suitable for performing filling, packing, or covering to a tooth. In Comparative Example 6, a dental composition having a total mass of the filler exceeding 90% by mass was tried to prepare by increasing the content of the filler by further adding the filler 2 to the formulation of the dental composition of Example 1. However, it was difficult to produce such a dental composition.

The dental compositions of Examples and Comparative Examples were measured and evaluated for the polymerization shrinkage stress, the flexural modulus, the flexural strength, the Vickers hardness, and the depth of photocuring, in the following manner.

### <Polymerization Shrinkage Stress>

On a glass plate having a thickness of 5.0 mm subjected to a sandblast treatment with 50 µm alumina powder, an adhesive material for dental ceramics ("Clearfil (registered trademark) Ceramic Primer Plus", available from Kuraray Noritake Dental Inc.) as a pretreatment material was coated and dried with air blowing.

On a stainless steel washer (inner diameter: of 5.3 mm and a thickness of 0.8 mm) having a release agent coated thereon separately provided, a dental adhesive material ("Clearfil (registered trademark) Megabond (registered trademark) 2 Bond", available from Kuraray Noritake Dental Inc.) was coated without leaving any excess, and after the washer was brough into close contact with the glass plate, the dental adhesive material was cured by irradiating the glass plate with light from the side that was not into contact with the washer with a dental light irradiator (Pencure 2000, available from Morita Corporation) in the normal mode for 10 seconds, so as to fix the washer and the glass plate to each other.

Subsequently, for fixing the glass plate and the dental composition to each other, a dental adhesive material ("Clearfil (registered trademark) Megabond (registered trademark) 2 Bond", available from Kuraray Noritake Dental Inc.) was coated inside the washer, and irradiated with light with a dental light irradiator (Pencure 2000, available from Morita Corporation) in the normal mode for 10 seconds. Thereafter, the dental composition in the form of paste produced in each of Examples and Comparative Examples was charged in the washer.

A stainless steel jig (diameter: 5 mm) subjected to a sandblast treatment was separately prepared, on which a dental adhesive material ("Clearfil (registered trademark) Megabond (registered trademark) 2 Bond", available from Kuraray Noritake Dental Inc.) was coated, and then irradiated with light from the coated side with a dental light irradiator (Pencure 2000, available from Morita Corporation) in the normal mode for 10 seconds. The dental composition in the washer was held between the stainless steel jig after light irradiation and the glass plate, and the excess dental composition was removed.

The dental composition charged in the washer was cured by irradiating the dental composition with light from the side of the glass plate with a dental LED light irradiator ("Pencure 2000", available from Morita Corporation) in the normal mode for 10 seconds, and the stress occurring 3 minutes after the start of light irradiation was designated as the polymerization shrinkage stress, and measured with a universal tester (Autograph AG-I 100 kN, available from Shimadzu Corporation) (n = 3). The arithmetic average value thereof was calculated and designated as the polymerization shrinkage stress of each of the dental compositions.

### <Flexural Modulus and Flexural Strength>

The flexural modulus and the flexural strength were evaluated by a flexural test according to ISO 4049:2019. The specific procedure was as follows.

The dental composition in the form of paste produced in each of Examples and Comparative Examples was charged in a stainless steel mold (2 mm in length × 25 mm in width × 2 mm in thickness), and the paste was pressed from above and below (on the surface of 2 mm × 25 mm) with glass slides. Subsequently, the paste was cured by irradiating the paste in the pressed state with light from above and below through the glass slides with a dental light irradiator (Pencure 2000, available from Morita Corporation) in the normal mode for 10 seconds on each of separate 5 positions per one side (i.e., irradiated for 50 seconds in total per one side), so as to provide a cured product. Five cured products were produced for each of Examples and Comparative Examples.

The cured product immersed in distilled water in a vessel was allowed to stand in a thermostat chamber set at 37°C for 24 hours, and then measured for the three-point flexural strength and the flexural modulus with a universal tester (Autograph AG-I 100 kN, available from Shimadzu Corporation) at a fulcrum distance of 20 mm and a crosshead speed of 1 mm/min (n = 5). The arithmetic average values thereof were calculated and designated as the flexural strength and the flexural modulus of the cured product of each of the dental compositions.

### <Vickers Hardness>

The dental composition produced in each of Examples and Comparative Examples was charged in a syringe, and the paste was charged in a polytetrafluoroethylene mold having a hole of 10 mm in diameter × 5 mm, and after pressing with a polypropylene film, irradiated with light with a dental light irradiator (Pencure 2000, available from Morita Corporation) brought into contact with the polypropylene film in the normal mode for 10 seconds, so as to produce a cured product (n = 1). The clear flat surface of the cured product was polished with #1500 polishing paper under a dry condition, and finally mirror polished with a diamond paste to provide a test piece. The surface of the test piece was measured for the Vickers hardness (Hv) with a micro hardness testing machine (HM-221, available from Mitutoyo Corporation) by applying a load of 200 g for 10 seconds. The measurement was performed at 5 positions in total including 1 position at the center and 4 positions around the center of the cured product, and the arithmetic average value of the measured values at the 5 positions was designated as the Vickers hardness of the dental composition of each of Examples and Comparative Examples.

### <Depth of Photocuring>

The depth of photocuring was evaluated according to ISO 4049:2019. The specific procedure was as follows. The dental composition produced in each of Examples and Comparative Examples was charged in a stainless steel mold (thickness: 12 mm, diameter: 4 mm). The upper and lower surfaces thereof each were covered with a film and a glass slide in this order and pressed therewith. The glass plate was removed from one side, and the dental composition was cured by irradiating with light from the surface thereof having the film pressed thereon, from which the glass plate had been removed, with a dental light irradiator ("Pencure 2000", available from Morita Corporation) for 10 seconds. The resulting cured product was taken out from the mold, from which the uncured portion was then wiped out, and the thickness of the remaining cured product was measured with a micrometer (available from Mitutoyo Corporation). The distance from the light irradiation surface to the cured product was calculated, and the half value of the calculated value was designated as the depth of photocuring (n = 5). The arithmetic average value thereof was calculated and designated as the depth of photocuring of the dental composition of each of Examples and Comparative Examples.

The measurement and evaluation results of dental composition of Examples and Comparative Examples are shown in Table 1 with the compositions thereof. In the columns "Composition" in Table 1, "-" means that the corresponding component was not blended.

### [Table 1]

**Table 1**

| | Class | Name of component/ Unit | Example 1 | Example 2 | Example 3 | Example 4 |
|---|---|---|---|---|---|---|
| Composition (part by mass) | Monomer (A-1) | 1-NMMA | 50 | 30 | 50 | 30 |
| | | 1-NMA | - | - | - | - |
| | Monomer (A-2) | UDMA | 50 | 70 | 50 | 55 |
| | | POB-MA | - | - | - | - |
| | | D-2.6E | - | - | - | - |
| | | 3G | - | - | - | 15 |
| | | THF-MA | - | - | - | - |
| | | MDP | - | - | - | - |
| | Polymerization initiator (B) | CQ | 0.2 | 0.2 | 0.2 | 0.2 |
| | | TMDPO | 0.3 | 0.3 | 0.3 | 0.3 |
| | Filler (C) | Filler 1 | 15 | 10 | - | - |
| | | Filler 2 | 250 | - | 265 | - |
| | | Filler 3 | - | 180 | - | 180 |
| | | Filler 4 | - | - | - | 80 |
| | Polymerization accelerator (D) | DABE | 0.3 | 0.3 | 0.3 | 0.3 |
| | Additive | BHT | 0.05 | 0.05 | 0.05 | 0.05 |
| Evaluation | Depth of photocuring | mm | 4.5 | 4.3 | 5.6 | 4.2 |
| | Flexural strength | MPa | 126 | 135 | 126 | 110 |
| | Flexural Modulus | GPa | 7.3 | 7.4 | 7.6 | 6.7 |
| | Vickers hardness | Hv | 38 | 45 | 40 | 36 |
| | Polymerization shrinkage stress | MPa | 7.3 | 7.4 | 7.3 | 7.3 |

**Table 1 (continued)**

| | Class | Name of component/ Unit | Example 5 | Example 6 | Example 7 | Example 8 |
|---|---|---|---|---|---|---|
| Composition (part by mass) | Monomer (A-1) | 1-NMMA | 30 | 30 | - | - |
| | | 1-NMA | - | - | 50 | 30 |
| | Monomer (A-2) | UDMA | 45 | 50 | 50 | 70 |
| | | POB-MA | - | - | - | - |
| | | D-2.6E | - | - | - | - |
| | | 3G | 25 | - | - | - |
| | | THF-MA | - | 20 | - | - |
| | | MDP | - | - | - | - |
| | Polymerization initiator (B) | CQ | 0.2 | 0.2 | 0.2 | 0.2 |
| | | TMDPO | 0.3 | 0.3 | 0.3 | 0.3 |
| | Filler (C) | Filler 1 | - | - | 15 | 10 |
| | | Filler 2 | 80 | 80 | 250 | - |
| | | Filler 3 | 180 | 180 | - | 180 |
| | | Filler 4 | - | - | - | - |
| | Polymerization accelerator (D) | DABE | 0.3 | 0.3 | 0.3 | 0.3 |
| | Additive | BHT | 0.05 | 0.05 | 0.05 | 0.05 |
| Evaluation | Depth of photocuring | mm | 5.8 | 5.5 | 4.8 | 4.4 |
| | Flexural strength | MPa | 121 | 131 | 125 | 131 |
| | Flexural Modulus | GPa | 8.9 | 9.3 | 7.2 | 7.1 |
| | Vickers hardness | Hv | 35 | 39 | 44 | 48 |
| | Polymerization shrinkage stress | MPa | 9.7 | 9.4 | 7.8 | 8.0 |

**Table 1 (continued)**

| | Class | Name of component/ Unit | Example 9 | Example 10 | Example 11 | Comparative Example 1 |
|---|---|---|---|---|---|---|
| Composition (part by mass) | Monomer (A-1) | 1-NMMA | 50 | 30 | 30 | - |
| | | 1-NMA | - | - | - | - |
| | Monomer (A-2) | UDMA | - | 50 | 55 | 50 |
| | | POB-MA | - | 20 | - | 50 |
| | | D-2.6E | 50 | - | - | - |
| | | 3G | - | - | 15 | - |
| | | THF-MA | - | - | - | - |
| | | MDP | - | - | 5 | - |
| | Polymerization initiator (B) | CQ | 0.2 | 0.2 | 0.2 | 0.2 |
| | | TMDPO | 0.3 | 0.3 | 0.3 | 0.3 |
| | Filler (C) | Filler 1 | 15 | 15 | - | 15 |
| | | Filler 2 | 250 | 250 | - | 250 |
| | | Filler 3 | - | - | 180 | - |
| | | Filler 4 | - | - | 80 | - |
| | Polymerization accelerator (D) | DABE | 0.3 | 0.3 | 0.3 | 0.3 |
| | Additive | BHT | 0.05 | 0.05 | 0.05 | 0.05 |
| Evaluation | Depth of photocuring | mm | 3.6 | 4.3 | 4.4 | 4.6 |
| | Flexural strength | MPa | 138 | 128 | 120 | 121 |
| | Flexural Modulus | GPa | 8.7 | 6.8 | 6.6 | 6.6 |
| | Vickers hardness | Hv | 46 | 35 | 34 | 32 |
| | Polymerization shrinkage stress | MPa | 9.4 | 6.8 | 7.5 | 6.8 |

**Table 1 (continued)**

| | Class | Name of component/ Unit | Comparative Example 2 | Comparative Example 3 | Comparative Example 4 | Comparative Example 5 |
|---|---|---|---|---|---|---|
| Composition (part by mass) | Monomer (A-1) | 1-NMMA | - | - | - | 50 |
| | | 1-NMA | - | - | - | - |
| | Monomer (A-2) | UDMA | 50 | 35 | - | 50 |
| | | POB-MA | 35 | 35 | - | - |
| | | D-2.6E | - | - | 70 | - |
| | | 3G | 15 | 30 | 30 | - |
| | | THF-MA | - | - | - | - |
| | | MDP | - | - | - | - |
| | Polymerization initiator (B) | CQ | 0.2 | 0.2 | 0.2 | 0.2 |
| | | TMDPO | 0.3 | 0.3 | 0.3 | 0.3 |
| | Filler (C) | Filler 1 | - | - | - | 15 |
| | | Filler 2 | - | - | - | 50 |
| | | Filler 3 | 180 | 180 | 180 | - |
| | | Filler 4 | 80 | 80 | 80 | - |
| | Polymerization accelerator (D) | DABE | 0.3 | 0.3 | 0.3 | 0.3 |
| | Additive | BHT | 0.05 | 0.05 | 0.05 | 0.05 |
| Evaluation | Depth of photocuring | mm | 4.6 | 4.6 | 5.5 | 7.2 |
| | Flexural strength | MPa | 110 | 87 | 115 | 110 |
| | Flexural Modulus | GPa | 6.2 | 6.7 | 6.9 | 3.8 |
| | Vickers hardness | Hv | 25 | 24 | 36 | 20 |
| | Polymerization shrinkage stress | MPa | unmeasurable | unmeasurable | 10.4 | unmeasurable |

As shown in Table 1, the cured products obtained by curing the dental compositions of Examples 1 to 11 had a large flexural strength and a high Vickers hardness. In particular, the dental compositions of Examples 2, 6, 8, and 9 provided cured products having a high flexural strength exceeding 130 MPa.

The cured products obtained by curing the dental compositions of Examples 5, 6, and 9 had a high flexural modulus of 8.7 GPa or more.

The cured products obtained by curing the dental compositions of Examples 2, 3, and 7 to 9 had a high Vickers hardness exceeding 40 Hv.

The cured products obtained by curing the dental compositions of Examples 1 to 4, 7, 10, and 11 were cured products having a polymerization shrinkage stress of less than 8.0 MPa.

As shown in Table 1, on the other hand, the cured products obtained by curing the dental compositions of Comparative Examples 1 to 4 containing no monomer (A-1) were inferior in at least one characteristic feature of the Vickers hardness and the polymerization shrinkage stress to the cured products of the dental composition of Examples. For example, Comparative Example 1 was inferior in the Vickers hardness to all Examples, and Comparative Example 4 was inferior in the polymerization shrinkage stress to all Examples.

The dental compositions of Comparative Examples 3 and 2 did not contain the monomer (A-1), and therefore the cured products thereof were brittle as compared to the dental composition of Example 4, making the measurement of the polymerization shrinkage stress difficult. These cured products were largely inferior in the Vickers hardness.

The dental composition of Comparative Example 5 had a content of the filler (C) of less than 50%, and therefore had a lower flexural modulus and Vickers hardness than the dental compositions of Examples, and the cured product thereof was brittle and was difficult to measure the polymerization shrinkage stress.

### Industrial Applicability

The dental composition of the present invention can be used for repairing any tooth. The dental composition of the present invention has a small difference in refractive index from the filler and a high depth of photocuring, and therefore the cured product thereof can be readily imparted with a high mechanical strength. Accordingly, the dental composition can be favorably applied to a dental composite resin, a self-adhesive dental composite resin, dental cement, and a dental core build-up material.

The present application is based on Japanese Patent Application filed on December 12, 2023 (Japanese Patent Application No. 2023-209300), the entire contents of which are incorporated herein by reference.

## Claims

1. A dental composition comprising a monomer (A), a polymerization initiator (B), and a filler (C),
the monomer (A) comprising a monomer (A-1) represented by the following formula (1) and a monomer (A-2) that is other than the monomer (A-1),
the dental composition comprising the filler (C) in an amount of 50 to 90% by mass based on 100% by mass of the dental composition: 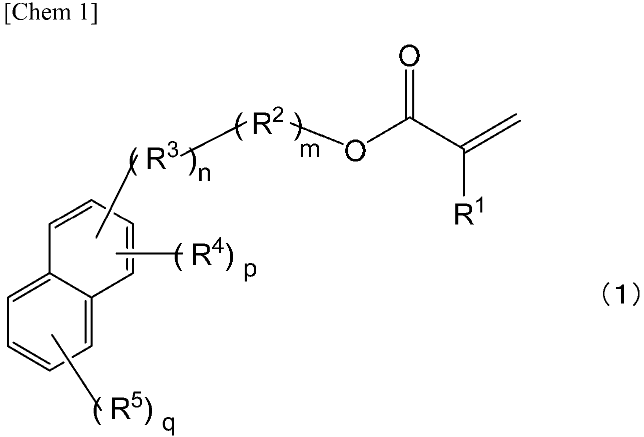 wherein,
R¹ represents a hydrogen atom or a methyl group, R² represents a divalent hydrocarbon group, R³ represents an oxygen atom or a sulfur atom,
R⁴ and R⁵ each independently represent a halogen atom, an alkyl group, or an alkoxy group, m represents 0 or 1, n represents 0 or 1, p represents 0 to 3, and q represents 0 to 4,
provided that
in the case where m represents 0, n represents 0,
in the case where p represents 2 or more, the multiple atoms or groups represented by R⁴ are the same atom or group or different atoms or groups, and
in the case where q represents 2 or more, the multiple atoms or groups represented by R⁵ are the same atom or group or different atoms or groups.

2. The dental composition according to claim 1, wherein the dental composition comprises the monomer (A-1) in an amount of 1 to 80 parts by mass per 100 parts by mass in total of the monomer (A-1) and the monomer (A-2).

3. The dental composition according to claim 1 or 2, wherein the monomer (A-1) is a monomer (A-1a) represented by the following formula (2): 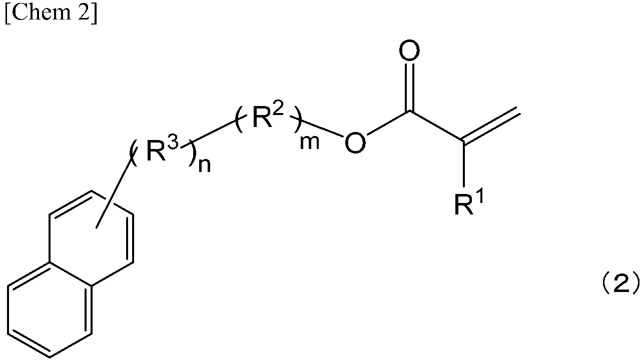 wherein, R¹, R², R³, m, and n have the same meanings as defined in the formula (1).

4. The dental composition according to claim 1 or 2, wherein the monomer (A-1) is 1-naphthylmethyl (meth)acrylate.

5. The dental composition according to claim 1 or 2, wherein the monomer (A-2) comprises a monomer having a urethane bond.

6. The dental composition according to claim 1 or 2, wherein the monomer (A-2) comprises a monomer having a (meth)acryloyl group.

7. The dental composition according to claim 1 or 2, wherein the dental composition has a content of a monomer having a bisphenol A skeleton of more than 0% by mass and 30% by mass or less, or does not contain a monomer having a bisphenol A skeleton.

8. The dental composition according to claim 1 or 2, wherein the dental composition is a dental composite resin.

9. The dental composition according to claim 1 or 2, wherein the dental composition is dental cement.

10. The dental composition according to claim 1 or 2, wherein the dental composition is a self-adhesive dental composite resin.

11. The dental composition according to claim 1 or 2, wherein the dental composition is a dental core build-up material.
